# EUROPEAN PATENT SPECIFICATION

(11) **EP 2 859 910 B1**
(45) Date of publication and mention of the grant of the patent: **25.03.2020**
(21) Application number: 13803698.3
(22) Date of filing: 11.06.2013
(51) Int. Cl.: A61M 37/00, A61N 2/00, A61N 1/32

(54) **MICRONEEDLE SHEET**
MIKRONADELBLECH
FEUILLE DE MICRO-AIGUILLES

(30) Priority: 12.06.2012 JP 2012133214
(43) Date of publication of application: 15.04.2015
(73) Proprietor: Hisamitsu Pharmaceutical Co., Inc., Tosu-shi, Saga 841-0017 (JP)
(72) Inventor: KOMINAMI Kazuya, Tsukuba-shi Ibaraki 305-0856 (JP); NISHIMURA Shinpei, Tsukuba-shi Ibaraki 305-0856 (JP); TOKUMOTO Seiji, Tsukuba-shi Ibaraki 305-0856 (JP); OGURA Makoto, Tsukuba-shi Ibaraki 305-0856 (JP); MATSUDO Toshiyuki, Tsukuba-shi Ibaraki 305-0856 (JP); YAMAMOTO Naoki, Tsukuba-shi Ibaraki 305-0856 (JP)
(74) Representative: Grünecker Patent- und Rechtsanwälte PartG mbB
(86) International application number: PCT/JP2013/066039
(87) International publication number: WO 2013/187392

(56) References cited:
- EP-A1- 1 360 935
- WO-A2-2007/127811
- JP-A- 2005 118 428
- JP-A- 2006 345 984
- JP-A- 2009 501 066
- JP-A- 2012 090 795
- US-A1- 2007 161 964

## Description

### Technical Field

A method for preparing a microneedle sheet with raised microneedles is provided. The method contains the features defined in claim 1.

### Background Art

Microneedles through which an active component is administered via the skin and apparatuses including the microneedles have been known. For example, a rotatable microstructure apparatus disclosed in Patent Literature 1 described below includes a curved base material and a roller structure with a plurality of microelements affixed to a first surface of the base material. The plurality of microelements have a predetermined size and a predetermined shape so as to allow the microstructure apparatus to penetrate the stratum corneum in the skin when the microstructure apparatus is placed on the skin and rolls in a predetermined direction.

US2007/161964 discloses a microneedle array device including a substantially planar substrate having an array of apertures and a plurality of microneedles projecting at an angle from the planar substrate. The microneedles have a base portion integrally connected to the substrate, a tip end portion distal to the base portion and body portion therebetween.

WO2007/127811 discloses a transdermal drug delivery system with microprojections for disrupting a body surface to an individual. At least some of the microprojections form groups in a micro-projection array.

EP 1360935 A1 discloses a continuous strip of testers, which are arranged in one row. Each tester has a needle in the front. The testers are exposed when the strip with the testers passes between rollers, while they are flexed away from the recess, in which they are provided in the strip.

### Citation List

### Patent Literature

Patent Literature 1: JP 2005-503210 A

### Summary of Invention

### Technical Problem

However, in the microstructure apparatus described in Patent Literature 1, the microelements are exposed on the roller, and thus, the needles may come into contact with or be caught on another object (for example, a user's skin or clothes) before the active component is applied to the skin via the microneedles. Thus, there has been a demand to ensure safety in handling of the microneedles.

### Solution to Problem

References to "embodiments" in the following and throughout the description which are not under the scope of the appended claims merely represent possible exemplary executions and are therefore not implied as being part of the present invention.

In particular, a method for preparing a microneedle sheet with raised microneedles is provided. The method has the features defined in claim 1. Further preferred embodiments are defined in the dependent claims.
In the following, some microneedle sheets are describes as further embodiments. The claims are related to a method for preparing a microneedle sheet with raised microneedles.

In such an embodiment, the microneedles are kept substantially along the principal surface of the sheet until the sheet is bent. This means that tips of the microneedles do not stick out from the principal surface before the microneedles are applied to the skin. Thus, the microneedles are prevented from coming into contact with or being caught on another object unless the microneedle sheet is applied to the skin. As a result, the safety in handling of the microneedles can be ensured.

In the microneedle sheet according to another embodiment, the sheet may be bent when a part of the sheet which does not contact the skin comes into contact with the skin, and the microneedles positioned in the part may rise from the principal surface. In this case, the microneedles rise from the principal surface immediately before the microneedles pierce the skin. Therefore, the safety in handling of the microneedles can be ensured.

Moreover, in the microneedle sheet according to yet another embodiment, the microneedles may be formed in lines extending along a direction orthogonal to a direction in which the sheet is guided to the skin, and the sheet may be guided to the skin to raise the microneedles line by line. Raising the microneedles line by line in this manner allows the microneedles on the sheet to be reliably raised before the microneedles pierce the skin.

In the microneedle sheet according to yet another embodiment, a maximum angle between the microneedle raised from the principal surface and a virtual line from a center of curvature of the sheet to a root of the microneedle may be larger than 90 degrees. In this case, the length of a part of the microneedle which pierces the skin increases, thus enhancing the cutaneous permeability of the active component.

In the microneedle sheet according to yet another embodiment, the maximum angle may be 95 to 130 degrees. In this case, the length of a part of the microneedle which pierces the skin increases, thus enhancing the cutaneous permeability of the active component.

In the microneedle sheet according to yet another embodiment, a ratio of a length of the microneedle to the radius of curvature of the sheet may be higher than 0.20. Setting the relation between the radius of curvature of the sheet and the length of the microneedle in this manner allows the microneedles to reliably pierce the skin.

In the microneedle sheet according to yet another embodiment, a puncture angle of the raised microneedle to the skin may be at least 34 degrees and smaller than 180 degrees.

In the microneedle sheet according to yet another embodiment, the sheet may be shaped like a band.

In the microneedle sheet according to yet another embodiment, the microneedle sheet can be used with another percutaneous absorption promotion technique, and the other percutaneous absorption promotion technique may include at least one of electricity, pressure, magnetic field, and ultrasonic wave.

### Advantageous Effects of Invention

According to an aspect of the present invention, the safety in handling of the microneedles can be ensured.

### Brief Description of Drawings

[Figure 1] Figure 1 is a perspective view of a puncture apparatus according to a first embodiment as seen from behind.
[Figure 2] Figure 2 is an enlarged perspective view of a bending portion depicted in Figure 1.
[Figure 3] Figure 3 is a plan view of a microneedle sheet according to the first embodiment.
[Figure 4] Figure 4 is a perspective view depicting a state in which the microneedle sheet is installed in the puncture apparatus.
[Figure 5] Figure 5 is a diagram depicting an application of the microneedle sheet to a skin.
[Figure 6] Figure 6 is an enlarged perspective view depicting a state in which microneedles have risen from a principal surface of the sheet.
[Figure 7] Figure 7 is a diagram schematically depicting the rise and puncture of the microneedle.
[Figure 8] Figure 8 is a perspective view of a microneedle sheet according to a second embodiment.
[Figure 9] Figure 9 is a diagram depicting an application of the microneedle sheet according to the second embodiment in which (a) is a perspective view, and (b) is a side view.
[Figure 10] Figure 10 is a diagram schematically depicting a manner of puncture in an example.
[Figure 11] Figure 11 is diagram schematically depicting the state of the microneedles after puncture in the example.
[Figure 12] Figure 12 is a graph depicting a relation between a rising angle and a puncture length (Example 1).
[Figure 13] Figure 13 is a graph depicting a relation between a needle length/radius of curvature ratio and the puncture length (Example 2).
[Figure 14] Figure 14 is a graph depicting a relation between the needle length/radius of curvature ratio and the puncture angle (Example 2).

### Description of Embodiments

Embodiments of the present invention will be described below in detail with reference to the drawings. In the description of the drawings, identical or similar elements are denoted by identical reference, and duplicate descriptions are omitted. In the following, some embodiments of microneedle sheets are described. The claim itself relates to a method.

### (First Embodiment)

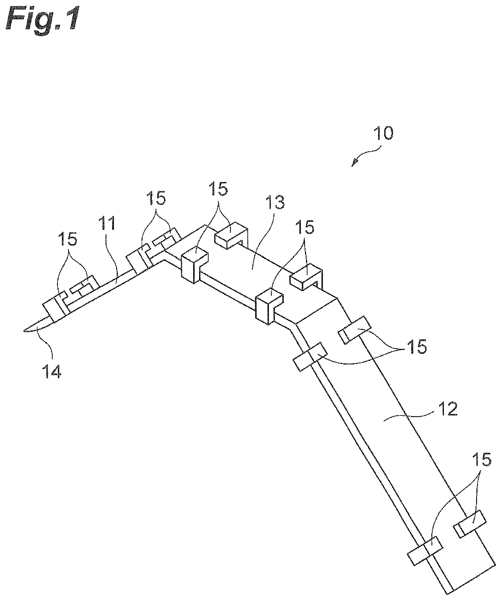

The structures of a puncture apparatus 10 and a microneedle sheet 20 according to a first embodiment will be described using Figures 1 to 3. The microneedle sheet 20 is an instrument with a large number of microneedles that pierce a skin. The puncture apparatus 10 is an auxiliary apparatus used to apply the microneedle sheet 20 to the skin.

First, the puncture apparatus 10 will be described. As depicted in Figure 1, the puncture apparatus 10 is produced by combining three thin plates, and is generally J-shaped as a whole. Specifically, the puncture apparatus 10 comprises a first plate (acting portion 11) that contacts the microneedle sheet 20 during use, a second plate (gripping portion 12) gripped by a user, and a third plate (intermediate portion 13) that connects the first plate and the second plate together. In the first embodiment, the gripping portion 12 is approximately double the acting portion 11 and the intermediate portion 13 in length. However, the lengths of these portions may be optionally set. Furthermore, in the first embodiment, the angle between the acting portion 11 and the intermediate portion 13 and the angle between the gripping portion 12 and the intermediate portion 13 are both obtuse but may also be optionally set. The acting portion 11, the gripping portion 12, and the intermediate portion 13 may be integrally molded. An example of a material for the plates is plastic such as an acrylic. However, the material is subject to no limitation, and for example, a metal or another type of resin may be used to produce the puncture apparatus 10.

In the specification, a side of the puncture apparatus 10 which faces upward (the upper side in Figure 1) when the puncture apparatus 10 is placed so that the intermediate portion 13 is positioned above the acting portion 11 and the gripping portion 12 is defined as an outside of the puncture apparatus 10. A side of the puncture apparatus 10 which faces downward (the lower side in Figure 1) in this case is defined as an inside of the puncture apparatus 10. When an active component is applied to the living body by using the puncture apparatus 10 and the microneedle sheet 20, the inside of the puncture apparatus 10 lies above the skin of the living body.

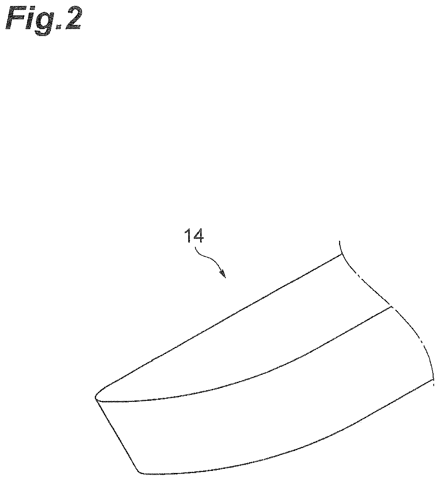

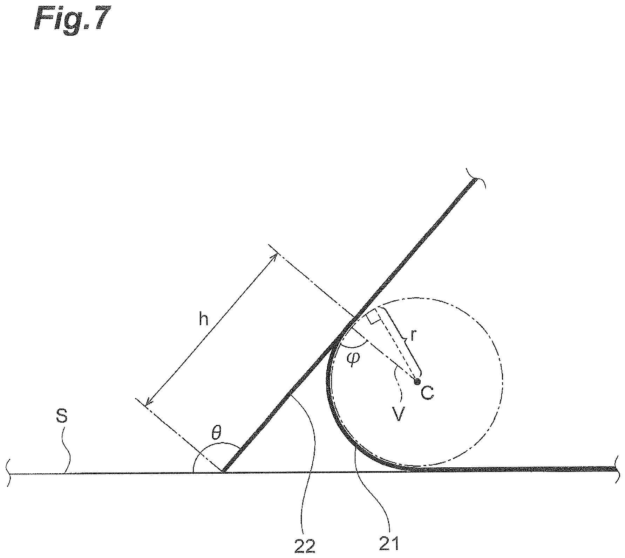

A leading end of the acting portion 11 functions as a bending portion 14 used to bend the microneedle sheet 20. As depicted in Figure 2, the bending portion 14 is shaped such that, toward a leading end of the bending portion 14, the inside thereof (first surface) is tapered toward the outside thereof (a second surface opposite to the first surface) and such that the leading end is round. However, the shape of the bending portion 14 is not limited to this. For example, the value of the radius of curvature r (see Figure 7) of the roundness of the leading end may be optionally set, a part of the bending portion 14 need not necessarily be tapered, or the leading end need not necessarily be rounded. Thus, a cornered bending portion like the leading end of the gripping portion 12 may be adopted.

A plurality of guide portions 15 is provided on the outside of the acting portion 11, the gripping portion 12, and the intermediate portion 13 to guide the microneedle sheet 20 to the bending portion 14 while holding the microneedle sheet 20 along an outer surface of the puncture apparatus 10. The guide portions 15 are arranged at predetermined intervals along a longitudinal direction of the acting portion 11, the gripping portion 12, and the intermediate portion 13. In the first embodiment, each of the guide portions 15 is formed using a pair of inverted L-shaped members disposed opposite each other in a width direction of the puncture apparatus 10. Of course, the structure of the guide portions 15 is subject to no limitation, and the guide portions 15 may be configured using any mechanical means or control means.

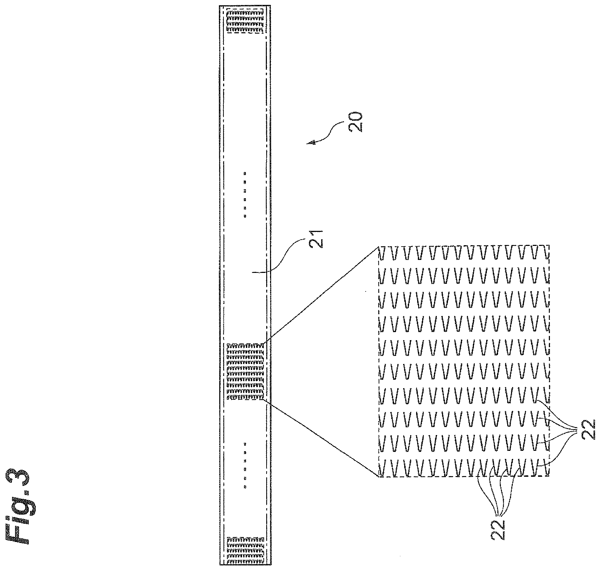

Now, the microneedle sheet 20 will be described. As depicted in Figure 3, the microneedle sheet 20 is shaped like a band, and has a plurality of microneedles 22 formed on the sheet substantially along a principal surface 21 of the sheet. The microneedles 22 are arranged in line in the longitudinal direction and in the width direction of the sheet. The tips of all the microneedles 22 unexceptionally face toward one end of the sheet (left direction in Figure 3).

Materials for the microneedle sheet 20 and the microneedles 22 are not limited. For example, the microneedle sheet 20 and the microneedles 22 may be produced using any of the materials such as stainless steel, polyethylene terephthalate (PET), another metal, another resin, a biodegradable material, a ceramic, and a bioabsorbable material. Alternatively, the microneedle sheet 20 and the microneedles 22 may be produced using a combination of these materials.

The microneedles 22 may be formed by etching. The microneedles 22 may be formed by punching a sheet with a chemical when the sheet is metal or by punching a sheet with a laser when the sheet is nonmetal. In these cases, a void is created around each of the microneedles 22. Of course, the microneedles 22 may be formed by means of a technique other than etching. As depicted in Figure 3, the microneedles 22 are triangular according to the first embodiment. However, the shape of the microneedle is subject to no limitation. In any case, the microneedles 22 need not be previously raised from the principal surface 21 of the sheet, allowing the microneedle sheet 20 to be easily and inexpensively manufactured.

The dimensions of the microneedle sheet 20 are also not limited. Specifically, a lower limit for thickness may be 5 µm or 20 µm, and an upper limit for thickness may be 1000 µm or 300 µm. A lower limit for length may be 0.1 cm or 1 cm, and an upper limit for length may be 50 cm or 20 cm. A lower limit for width may be 0.1 cm or 1 cm, and an upper limit for width may be 60 cm or 30 cm. The lower limits for the length and width of the microneedle sheet 20 are set taking into account the amount of active component administered. The upper limits for the length and width are set taking the size of the living body into account.

Parameters for the microneedles 22 are also not limited. Specifically, a lower limit for the height of the needle may be 10 µm or 100 µm, and an upper limit for the height of the needle may be 10000 µm or 1000 µm. A lower limit for the density of the needles may be 0.05/cm² or 1/cm², and an upper limit for the density of the needles may be 10000/cm² or 5000/cm². The lower limit for the density results from conversion of the number and area of needles through which 1 mg of active component can be administered. The upper limit for the density is a limit value set taking the shape of the needle into account.

The dimensions of the puncture apparatus 10 may be determined in accordance with the dimensions of the microneedle sheet 20. For example, the length of the puncture apparatus 10 (the sum of lengths of the acting portion 11, the intermediate portion 13, and the gripping portion 12) along an outer surface thereof may be equal to or smaller than the length of the microneedle sheet 20.

Possible methods for preparing an active component applied to the skin include a technique for pre-coating the active component on the microneedle sheet 20 itself, a technique for applying the active component onto the skin before the microneedles 22 pierce the skin, and a technique for applying the active component onto the skin after the microneedles 22 pierce the skin. When the active component is pre-coated on the microneedle sheet 20, a coating liquid with a predetermined viscosity is preferably applied all over the sheet to as uniform a thickness as possible. Since the microneedles 22 lie along the principal surface 21, such application can easily achieved. The coating may be performed using the principle of screen printing or any other method. When a biodegradable sheet is used, the active component may be contained in the sheet itself.

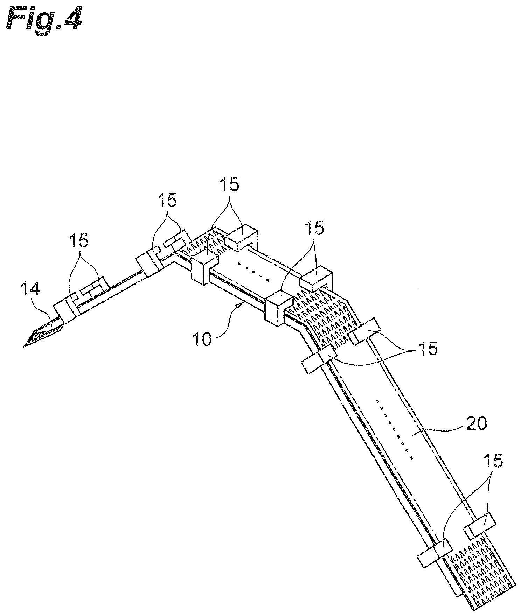

Now, using Figures 4 to 7, a method for using the puncture apparatus 10 and the microneedle sheet 20 according to the first embodiment will be described. First, as depicted in Figure 4, the user passes the microneedle sheet 20 through the plurality of guide portions 15 and moves one end of the microneedle sheet 20 to the vicinity of the bending portion 14, so as to hold the microneedle sheet 20 along the outer surface of the puncture apparatus 10. At this time, the user sets the microneedle sheet 20 in the puncture apparatus 10 so that the tips of the microneedles 22 face toward the bending portion 14. Subsequently, the user applies the one end of the microneedle sheet 20 to the skin (more specifically, to or near an edge of a site where the active component is to be applied).

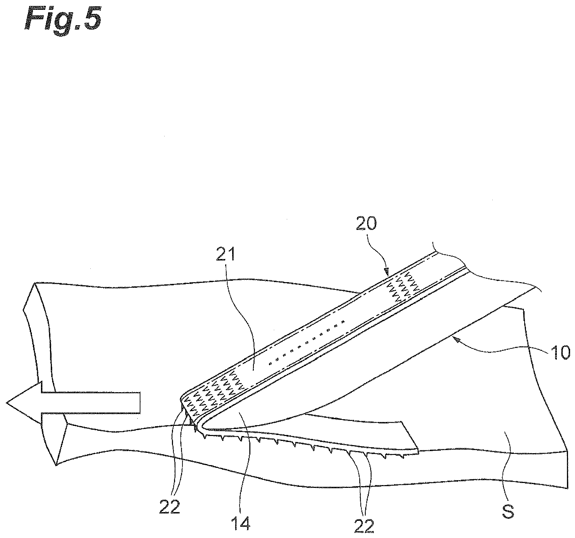

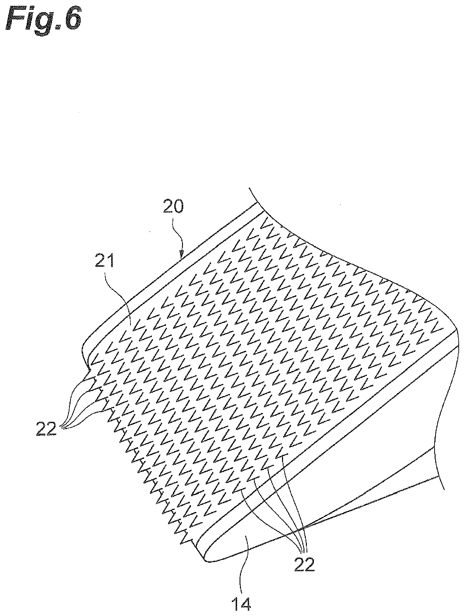

Subsequently, as depicted Figure 5, the user moves the puncture apparatus 10 on the skin S so as to bend the microneedle sheet 20 at an acute angle, thus moving the bending portion 14 forward. This operation allows the microneedle sheet 20 to be guided to the bending portion 14, and a part of the microneedle sheet 20 which has reached the bending portion 14 is bent along the bending portion 14. Then, as depicted in Figure 6, the microneedles 22 positioned in the bent portion rise from the principal surface 21 of the sheet. The raised microneedles 22 pierce the skin S as depicted in Figure 5.

In this regard, the microneedles 22 that rise at a time are a line of microneedles 22 along a width direction (orthogonal to a direction in which the microneedle sheet 20 is guided). The angle between the each of the raised microneedles 22 and the principal surface 21 is obviously larger than 0 degrees and smaller than 180 degrees.

A puncture angle θ (the angle between each of the microneedles 22 and the skin S) at which the microneedles 22 with a height h raised from the principal surface 21 as depicted in Figure 7 pierce the skin is also larger than 0 degrees and smaller than 180 degrees. A lower limit for the puncture angle may be 20 degrees, 34 degrees, or 40 degrees. An upper limit for the puncture angle may be 160 degrees, 140 degrees, or 100 degrees. Immediately after piercing the skin, the microneedles 22 are further pushed into the body by the puncture apparatus 10.

The value r in Figure 7 is indicative of the radius of curvature at the leading end of the bending portion 14. The maximum angle ϕ between the microneedle 22 raised from the principal surface 21 by folding back the microneedle sheet 20 and a virtual line V from the center of curvature C to the root of the microneedle is larger than 90 degrees. For example, the maximum angle may be in a range between 95 and 130 degrees or in a range between 95 and 120 degrees.

Increasing the ratio (h/r) of a needle length h to the radius of curvature r above 0.20 enables the microneedles 22 to reliably pierce the skin S.

When the user moves the puncture apparatus 10 on the skin by a desired distance, the plurality of microneedles 22 within the range of the distance pierce the skin. Thus, the user can adjust the area of application of the microneedle sheet 20 to administer a desired amount of active component.

As described above, according to the first embodiment, the microneedles 22 remain extending substantially along the principal surface 21 of the sheet until the microneedle sheet 20 is bent by the bending portion 14 of the puncture apparatus 10. This means that the tips of the microneedles 22 do not stick out from the principal surface 21. Thus, unless the puncture apparatus 10 is used, the microneedles 22 are prevented from coming into contact with or being caught on another object (for example, the user's skin or clothes). As a result, the safety in handling of the microneedles 22 can be ensured. For example, the user can safely store and convey the microneedle sheet 20 and prepare the microneedle sheet 20 before use.

In this regard, the microneedle sheet 20 is thin and flexible and can thus be applied to the skin in accordance with the shape of the living body. As a result, the active component can be efficiently administered.

Furthermore, instead of exerting an impact on the microneedle sheet 20, the puncture apparatus 10 raises and pushes the microneedles 22 into the skin to allow the needles 22 to pierce the skin. Thus, the active component can be administered to the patient without offering a feeling of fear to the patient.

In the first embodiment, the guide portions 15 guide the microneedle sheet 20 to the bending portion 14 to gradually raise the microneedles 22, thus allowing adjustment of the range of application of the microneedles 22 to the skin. Furthermore, such guidance of the microneedle sheet 20 can be achieved by an easy operation of moving the bending portion 14 forward on the skin. Moreover, since the microneedles 22 rise one line at a time, each of the microneedles 22 on the microneedle sheet 20 can be reliably raised and pierce the skin.

In the first embodiment, the leading end of the bending portion 14 is round. Thus, when the microneedle sheet 20 is bent, pressure from the bending portion 14 is not concentrated at a particular position on the sheet 20. Therefore, when applied, the microneedle sheet can be more reliably prevented from being damaged. Furthermore, since the bending portion 14 is tapered as described above, the user can smoothly move the bending portion 14 forward on the skin. As a result, the microneedle sheet 20 can be easily applied.

### (Second Embodiment)

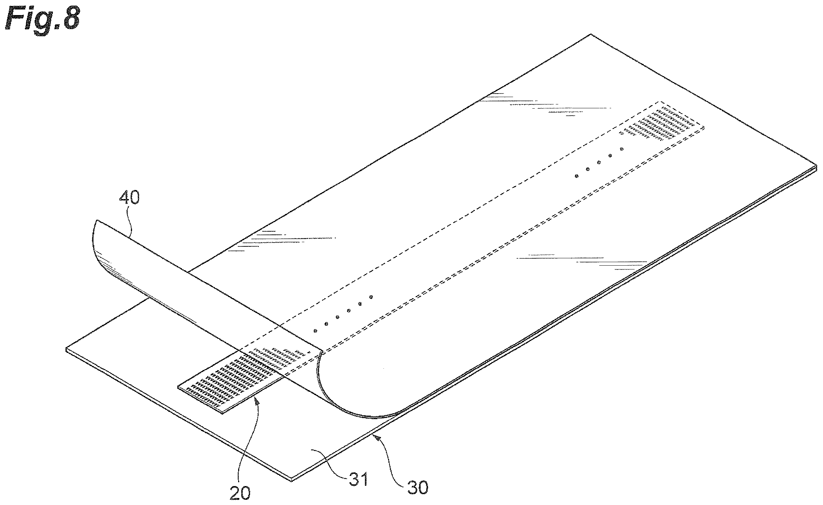

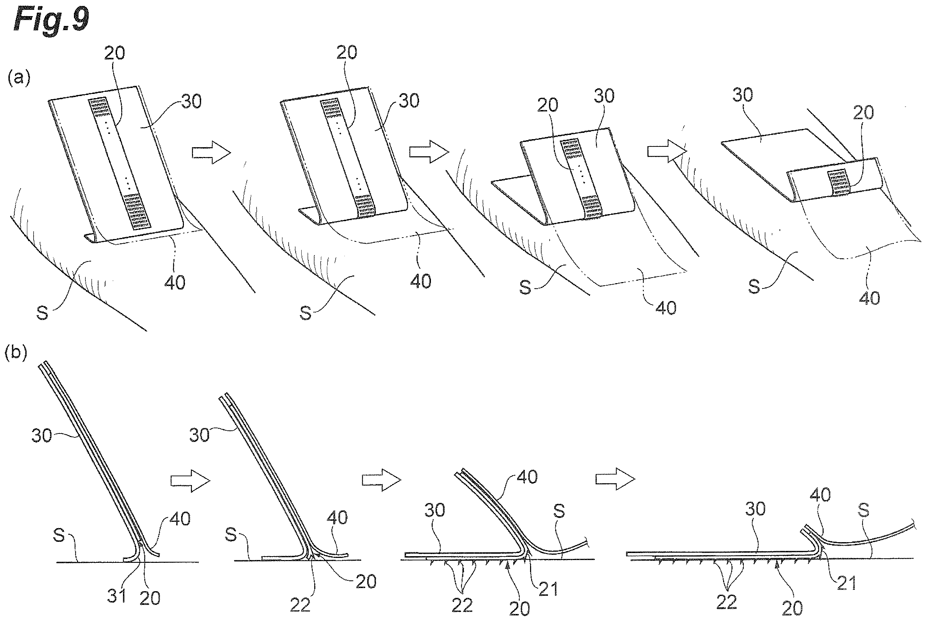

Using Figure 8 and Figure 9, a microneedle sheet 20 according to a second embodiment will be described. As depicted in Figure 8, the microneedle sheet 20 according to the second embodiment is attached to an affixing surface 31 of a base material 30 which comes into contact with the skin when the microneedle sheet 20 is applied. The microneedle sheet 20 is protected by a release film 40. In other words, the microneedle sheet 20 is sandwiched between the base material 30 and the release film 40. When the microneedle sheet 20 is applied to the skin, the release film 40 is released from the base material 30 and the sheet 20.

The base material 30 and the release film 40 are the same in shape and size. The length and width of these two members are larger than the length and width, respectively, of the microneedle sheet 20. Of course, the shapes of the base material 30 and the release film 40 may be optionally determined. Furthermore, the sizes of the base material 30 and the release film 40 may be optionally set. For example, the base material 30 and release film 40 may be used each of which has the same length and width as the length and width of the microneedle sheet 20.

Using Figure 9, a method for using the microneedle sheet 20 according to the second embodiment will be described. First, the user releases one end of the release film 40 to expose a part of the affixing surface 31 of the base material 30, and affixes the exposed part to the skin S. Subsequently, the user affixes the base material 30 with the microneedle sheet 20 anchored thereto to the skin S while gradually releasing the release film 40, so as to bend the microneedle sheet 20 at an acute angle.

Then, microneedles 22 positioned in a bent portion of the microneedle sheet 20 rise line by line from a principal surface 21 of the sheet. The raised microneedles 22 pierce the skin S one after another. The angle between each of the raised microneedles 22 and the principal surface 21 and the puncture angle at which the microneedles 22 pierce the skin are similar to the corresponding angles in the first embodiment. In the second embodiment, the user can adjust the area of application of the microneedle sheet 20 to administer a desired amount of active component.

The second embodiment allows effects similar to the effects of the first embodiment to be exerted. Specifically, the microneedles 22 remain extending substantially along the principal surface 21 of the sheet until the microneedle sheet 20 is bent directly by the user. Thus, unless the microneedle sheet 20 is applied to the skin, the microneedles 22 are prevented from coming into contact with or being caught on another object. As a result, the safety in handling of the microneedles 22 can be ensured. Furthermore, the microneedle sheet 20 can be applied to the skin in accordance with the shape of the living body, and the microneedles 22 can be raised line by line to allow reliable puncture, as is the case with the first embodiment.

### Examples

The present invention will be specifically described below based on examples. However, the present invention is not limited to the examples.

### (Example 1)

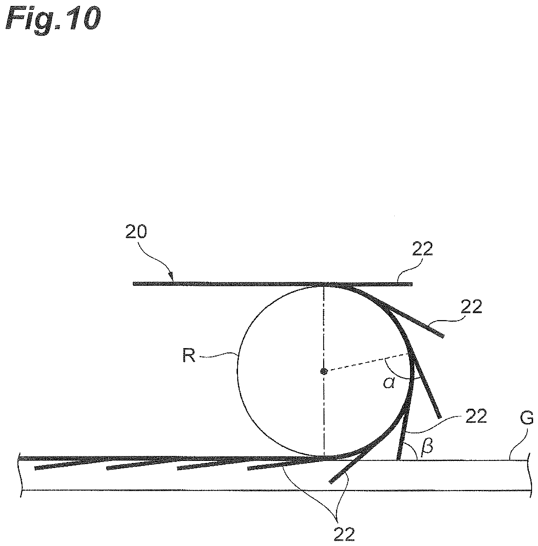

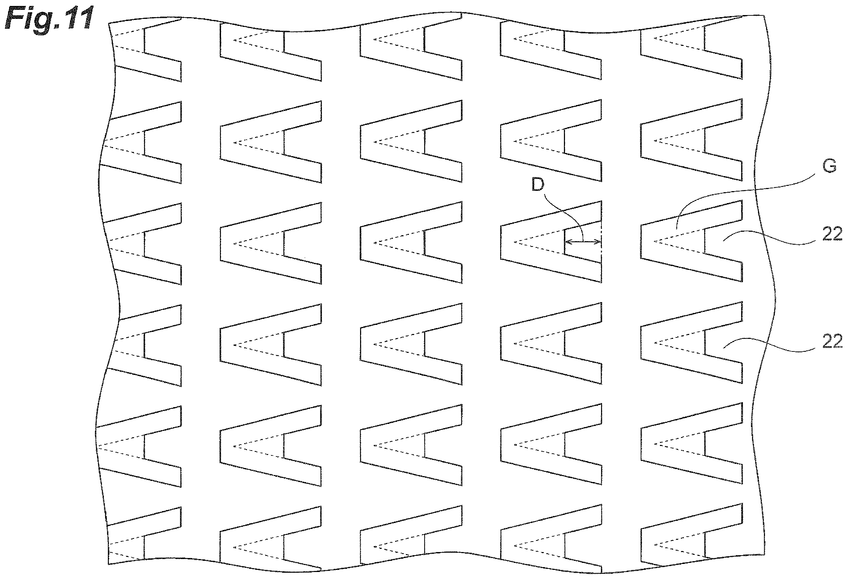

Experiments were conducted in which a gel sheet was used as an alternative to the skin and in which the microneedles of the microneedle sheet pierce the gel sheet. Specifically, a cylindrical thin rod (hereinafter referred to as a "cylindrical rod") R was placed along the width direction of the microneedle sheet 20. The microneedle sheet 20 was folded back using the cylindrical rod R to raise triangular microneedles 22. Moreover, the cylindrical rod R was moved along an upper surface of the gel sheet G to allow the microneedles 22 to pierce the gel sheet G. Figure 10 schematically depicts this manner of puncture. Furthermore, Figure 11 schematically depicts the state of the microneedles 22 after puncture.

In Example 1, the relation between a rising angle and a puncture length was observed. The rising angle refers to the maximum angle between the microneedle raised from the principal surface of the microneedle sheet by folding back the microneedle sheet and a virtual line from the center of rotation of the cylindrical rod to a root of the microneedle. The rising angle is synonymous with an angle ϕ in Figure 7. In Figure 10, the rising angle is denoted by α. On the other hand, the puncture length is the mean value of the lengths of the microneedles having entered the gel sheet. The puncture length of each microneedle is obtained by subtracting the length of the exposed part of the microneedle (a length depicted by reference numeral D in Figure 11) from the overall length of the microneedle.

Three types of microneedle sheets were prepared which were 5 µm, 10 µm, and 20 µm, respectively, in thickness. The lengths of the three microneedle sheets were all 500 µm. On the other hand, two types of cylindrical rods were prepared which were 1.2 mm and 2.0 mm, respectively, in diameter. Thus, the combination of the microneedle sheet and the cylindrical rod had six patterns.

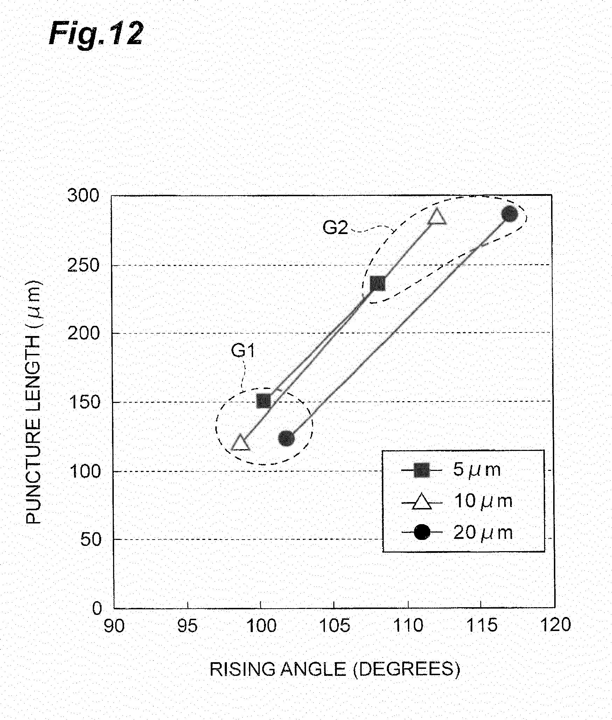

Figure 12 depicts the relation between the rising angle and the puncture length resulting from experiments performed on the six patterns. The abscissa of the graph indicates the rising angle (degrees), and the ordinate of the graph indicates the puncture length (µm). As depicted in the graph, the rising angle exceeded 95 degrees in all cases. Furthermore, the graph indicates that, for all of the three types of microneedle sheets, both the rising angle and the puncture length are larger with the cylindrical rod with a diameter of 1.2 mm (group G2 in Figure 12) than with the cylindrical rod with a diameter of 2.0 mm (group G1 in Figure 12). Since each of the microneedles is triangular, the length of an incision in the skin surface increases consistently with puncture length, with the result of an increase in the cutaneous permeability of the active component. Therefore, a larger rising angle is more preferable.

### (Example 2)

In this example, the relation between the ratio of the length of the microneedle to the radius of curvature (hereinafter referred to as the "needle length/radius of curvature ratio") and the puncture angle and puncture length was observed. As is the case with Example 1, in this example, experiments were performed using a gel sheet as an alternative to the skin. The manner of raising triangular microneedles and the puncture method were similar to the corresponding manner and method in Example 1 (see Figures 10 and 11). The radius of curvature is half the diameter of the cylinder. In Figure 10, the puncture angle is denoted by β.

For the microneedle sheet, six patterns were prepared which were combinations of two types of thickness (10 µm and 20 µm) and three type of needle length (200 µm, 250 µm, and 500 µm). Two types of cylindrical rods were prepared which were 1.2 mm and 2.0 mm, respectively, in diameter. The needle length/radius of curvature ratio involves the combinations of three types of needle length and two types of radius of curvature (0.6 mm and 1.0 mm) and thus has a total of six patterns as illustrated below.

0.5/0.6 ≈ 0.83
0.5/1.0 = 0.50
0.25/0.6 ≈ 0.42
0.2/0.6 ≈ 0.33
0.25/1.0 = 0.25
0.2/1.0 = 0.20

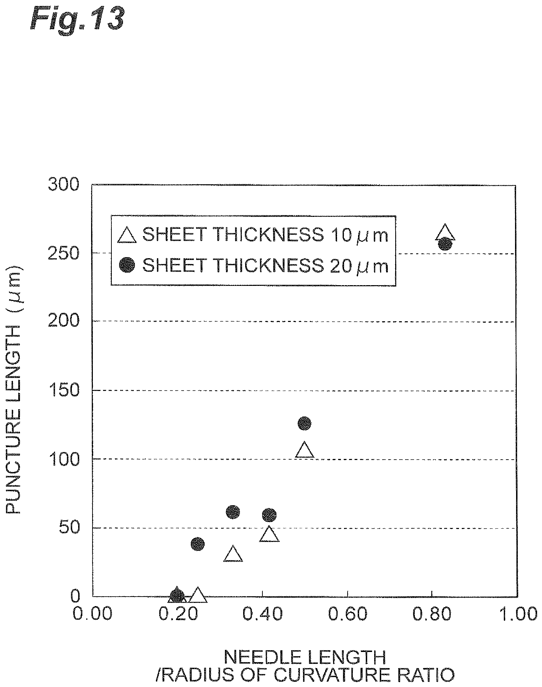

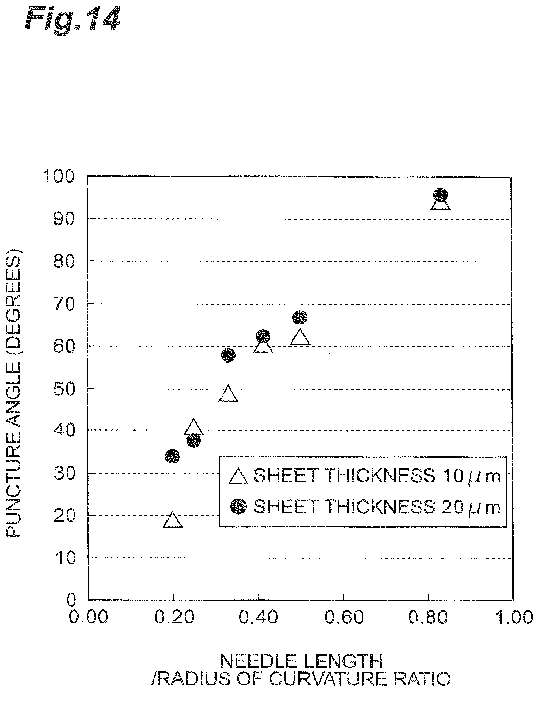

Figure 13 depicts the relation between the needle length/radius of curvature ratio and the puncture length (µm) resulting from experiments performed on all the combinations (12 patterns) of the microneedle sheet and the cylindrical rod. Furthermore, Figure 14 depicts the relation between the needle length/radius of curvature ratio and the puncture angle (degrees) obtained for the 12 patterns. Moreover, Table 1 illustrated below also depicts the results illustrated in the two graphs.

**[Table 1]**

| Cylinder diameter (mm) | Needle length (mm) | Radius of curvature | Needle length/radius of curvature | Puncture length (µm) (thickness: 10 µm) | Puncture angle (degrees) (thickness: 10 µm) | Puncture length (µm) (thickness: 20 µm) | Puncture angle (degrees) (thickness: 20 µm) |
|---|---|---|---|---|---|---|---|
| φ1.2 | 0.5 | 0.6 | 0.83 | 266.0 | 93.8 | 256.7 | 95.6 |
| φ2 | 0.5 | 1 | 0.50 | 106.67 | 61.56 | 125.67 | 66.68 |
| φ1.2 | 0.25 | 0.6 | 0.42 | 45.33 | 59.93 | 59.33 | 62.15 |
| φ1.2 | 0.2 | 0.6 | 0.33 | 31.33 | 48.34 | 61.67 | 57.58 |
| φ2 | 0.25 | 1 | 0.25 | 0.00 | 40.63 | 38.00 | 37.68 |
| φ2 | 0.2 | 1 | 0.20 | 0.00 | 18.64 | 0.0 | 33.7 |

The results depicted in Figures 13 and 14 and Table 1 indicate that puncture is enabled when the needle length/radius of curvature ratio is higher than 0.20. The puncture angle was equal to or larger than 34 degrees when the puncture was enabled.

The present invention has been described in detail based on the embodiments thereof. However, the present invention is not limited to the above-described embodiments. Many variations may be made to the present invention within a scope not departing from that of the appended claims.

In connection with the above-described first embodiment, when an element corresponding to the bending portion 14 is provided, the shape and structure of the puncture apparatus are subject to no limitation. For example, the puncture apparatus may be shaped like a single linear rod. Alternatively, the puncture apparatus may include any mechanical, electrical, or electronic structure or control means.

The shape of the microneedle sheet is not limited to a band-like shape. For example, the microneedle sheet may be a rectangle with a length and a width that are substantially the same, or a circle or an ellipse. In connection with the above-described first embodiment, means with functions similar to the functions of the guide portions 15, that is, a structure or control means that guides the microneedle sheet to the bending portion, may be omitted depending on the shape of the microneedle sheet.

In connection with the above-described second embodiment, the microneedle sheet 20 may be independently used, and thus, the base material 30 and the release film 40 are not indispensable.

The microneedle sheet according to the present disclosure can be used with another percutaneous absorption promotion technique such as electricity (iontophoresis), pressure, magnetic field, or ultrasound (sonophoresis). The use of the microneedle sheet with such another technique enables a further increase in the amount of drug absorbed.

### Reference Signs List

10 ... puncture apparatus, 11 ... acting portion, 12 ... gripping portion, 13 ... intermediate portion, 14 ... bending portion, 15 ... guide portion, 20 ... microneedle sheet, 21 ... principal surface, 22 ... microneedle, 30 ... base material, 31 ... affixing surface, 40 ... release film.

## Claims

1. Method for preparing a microneedle sheet (20) with raised microneedles (22), comprising the steps of:
providing a microneedle sheet (20) comprising a plurality of microneedles (22) formed on a sheet (20) and extending along a principal surface of the sheet (20),
**characterized in that**
the microneedles (21) are risen from the principal surface (21) by bending the sheet (20), and
the sheet (20) is configured to bend when a part of the sheet which does not contact the skin comes into contact with the skin, and the microneedles (22) positioned in the part rise from the principal surface (21).

2. The method according to claim 1, **characterized in that**
the plurality of microneedles (22) are arranged in line in a longitudinal direction and in a width direction of the sheet,
the microneedles (22) are formed in lines extending along the width direction, and
the sheet (20) is configured to be guided to the skin to raise the microneedles (21) line by line.

3. The method according to claim 1 or 2, **characterized in that** a puncture angle of the raised microneedle (22) to the skin is at least 34 degrees and smaller than 180 degrees.

4. The method according to any one of claims 1 to 3, **characterized in that** a maximum angle between the microneedle (22) raised from the principal surface (21) and a virtual line from a center of curvature of the sheet (20) to a root of the microneedle (22) is larger than 90 degrees.

5. The method according to claim 4, wherein the maximum angle is 95 to 130 degrees.

6. The method according to any one of claims 1 to 5, wherein a ratio of a length of the microneedle (22) to the radius of curvature of the sheet (20) is higher than 0.20.

7. The method according to any one of claims 1 to 6, wherein the microneedle sheet (20) is usable with another percutaneous absorption promotion technique, and
the other percutaneous absorption promotion technique includes at least one of electricity, pressure, magnetic field, and ultrasound.

## Patentansprüche

1. Verfahren zur Herstellung eines Mikronadelbogens (20) mit erhabenen Mikronadeln (22), das die folgenden Schritte umfasst:
Bereitstellen eines Mikronadelbogens (20), der eine Vielzahl von Mikronadeln (22) umfasst, die auf einem Bogen (20) ausgebildet sind und sich entlang einer Hauptoberfläche des Bogens (20) erstrecken,
**dadurch gekennzeichnet, dass**
die Mikronadeln (21) von der Hauptoberfläche (21) durch Biegen des Bogens (20) angehoben werden, und
der Bogen (20) so konfiguriert ist, dass es sich biegt, wenn ein Teil des Bogens, der nicht mit der Haut in Kontakt kommt, mit der Haut in Kontakt kommt, und die in dem Teil positionierten Mikronadeln (22) von der Hauptoberfläche (21) aufsteigen.

2. Das Verfahren nach Anspruch 1, **dadurch gekennzeichnet, dass**
die Vielzahl von Mikronadeln (22) in Längsrichtung und in Breitenrichtung des Bogens in einer Linie angeordnet sind,
die Mikronadeln (22) in Linien ausgebildet sind, die sich in der Breitenrichtung erstrecken, und
der Bogen (20) so konfiguriert ist, dass er zur Haut geführt wird, um die Mikronadeln (21) Linie für Linie anzuheben.

3. Verfahren nach Anspruch 1 oder 2, **dadurch gekennzeichnet, dass** ein Einstichwinkel der angehobenen Mikronadeln (22) zur Haut mindestens 34 Grad und kleiner als 180 Grad ist.

4. Verfahren nach einem der Ansprüche 1 bis 3, **dadurch gekennzeichnet, dass** ein maximaler Winkel zwischen der Mikronadel (22), die von der Hauptoberfläche (21) angehoben ist, und einer virtuellen Linie von einem Krümmungsmittelpunkt des Bogens (20) zu einer Wurzel der Mikronadel (22) größer als 90 Grad ist.

5. Verfahren nach Anspruch 4, wobei der maximale Winkel 95 bis 130 Grad beträgt.

6. Verfahren nach einem der Ansprüche 1 bis 5, wobei das Verhältnis der Länge der Mikronadel (22) zum Krümmungsradius des Bogens (20) größer als 0,20 ist.

7. Verfahren nach einem der Ansprüche 1 bis 6, wobei der Mikronadelbogen (20) mit einer anderen Technik zur Förderung der perkutanen Absorption verwendbar ist, und
die andere Technik zur Förderung der perkutanen Absorption umfasst mindestens eine der Techniken Elektrizität, Druck, Magnetfeld und Ultraschall.

## Revendications

1. Procédé de préparation d'une feuille (20) à microaiguilles avec des microaiguilles (22) dressées, comprenant les étapes de :
fourniture d'une feuille (20) à microaiguilles comprenant une pluralité de microaiguilles (22) formées sur une feuille (20) et s'étendant le long d'une surface principale de la feuille (20),
**caractérisé en ce que**
les microaiguilles (21) sont dressées depuis la surface principale (21) par flexion de la feuille (20), et
la feuille (20) est configurée pour fléchir lorsqu'une partie de la feuille qui n'entre pas en contact avec la peau entre en contact avec la peau, et les microaiguilles (22) positionnées dans la partie se dressent depuis la surface principale (21).

2. Procédé selon la revendication 1, **caractérisé en ce que**
la pluralité des microaiguilles (22) sont disposées en ligne dans un sens longitudinal et dans un sens de la largeur de la feuille,
les microaiguilles (22) sont formées en lignes s'étendant le long du sens de la largeur, et
la feuille (20) est configurée pour être guidée vers la peau pour dresser les microaiguilles (21) ligne par ligne.

3. Procédé selon la revendication 1 ou 2, **caractérisé en ce qu'**un angle de perforation de la microaiguille (22) dressée vers la peau est d'au moins 34 degrés et inférieur à 180 degrés.

4. Procédé selon l'une quelconque des revendications 1 à 3, **caractérisé en ce qu'**un angle maximal entre la microaiguille (22) dressée depuis la surface principale (21) et une ligne virtuelle depuis un centre de courbure de la feuille (20) vers une racine de la microaiguille (22) est supérieur à 90 degrés.

5. Procédé selon la revendication 4, dans lequel l'angle maximal est de 95 à 130 degrés.

6. Procédé selon l'une quelconque des revendications 1 à 5, dans lequel un rapport d'une longueur de la microaiguille (22) au rayon de courbure de la feuille (20) est supérieur à 0,20.

7. Procédé selon l'une quelconque des revendications 1 à 6, dans lequel la feuille (20) à microaiguilles peut être utilisée avec une autre technique de renforcement de l'absorption percutanée, et
l'autre technique de renforcement de l'absorption percutanée comprend au moins l'une parmi l'électricité, la pression, le champ magnétique, et l'ultrason.
